# FASCICULE DE BREVET EUROPEEN

(11) **EP 1 259 476 B1**
(45) Date de publication et mention de la délivrance du brevet: **05.10.2005**
(21) Numéro de dépôt: 01909919.1
(22) Date de dépôt: 28.02.2001
(51) Int. Cl.: C07C 209/74, C07D 231/12

(54) **CHLORATION D'UNE ANILINE EN ORTHO DANS UN MILIEU FLUORHYDRIQUE**
ORTHOCHLORIERUNG EINES ANILINS IN EINEM FLUORWASSERSTOFFMEDIUM
CHLORINATION OF AN ANILINE IN ORTHO IN A HYDROFLUORIC MEDIUM

(30) Priorité: 01.03.2000 FR 0002628
(43) Date de publication de la demande: 27.11.2002
(73) Titulaire: RHODIA CHIMIE, 92512 Boulogne Billancourt Cedex (FR)
(72) Inventeur: KEMPF, Hubert, F-30340 Salindres (FR); GUIDOT, Gilbert, F-30100 Ales (FR); SAINT-JALMES, Laurent, F-69330 Meyzieu (FR)
(74) Mandataire: Ricalens, François
(86) Numéro de dépôt international: PCT/FR2001/000578
(87) Numéro de publication internationale: WO 2001/064623

(56) Documents cités:
- US-A- 4 748 277
- US-A- 5 145 958
- US-A- 5 471 002

## Description

La présente invention vise un procédé de synthèse d'une aniline chlorée sur le noyau et comportant au moins un atome de carbone d'hybridation sp³ à la fois perhalogéné et porteur d'un atome de fluor. Il vise plus particulièrement la chloration d'une aniline précurseur de ladite aniline chlorée dans un milieu fluorhydrique.

Les molécules fluorées jouent un rôle grandissant dans la pharmacie et dans l'agrochimie. La synthèse des dérivés fluorés est souvent délicate car les réactions de fluoration directe sont le plus souvent trop violentes et trop sélectives pour que l'on puisse les mettre en oeuvre. Aussi, la synthèse des dérivés fluorés met souvent en oeuvre les échanges de divers substituants avec le fluor, et notamment les échanges d'halogènes plus lourds avec le fluor.

Le réactif le plus utilisé et le moins cher pour réaliser l'échange est indéniablement l'acide fluorhydrique en phase liquide.

L'acide fluorhydrique présente diverses propriétés qui sortent de l'ordinaire et rend les réactions faites au sein d'un milieu fluorhydrique peu prédictibles et en tous cas différentes des résultats obtenus avec les techniques usuelles.

En particulier, l'acide fluorhydrique liquide est un milieu particulièrement agressif et où de nombreuses associations par liaison hydrogène ont lieu ; d'autre part, l'acide fluorhydrique forme avec les amines, et notamment avec les amines faibles et avec les hétérocycles aromatiques, des complexes extrêmement stables qu'il est difficile de traiter et pour redonner l'amine libre et l'acide fluorhydrique.

Le plus souvent, une aniline se lie avec quatre molécules d'acide fluorhydrique qu'il sera nécessaire de neutraliser pour libérer l'amine.

Cette neutralisation est particulièrement coûteuse et conduit à rejeter des sels de l'acide fluorhydrique dont le rejet est en général sévèrement réglementé.

A ce jour, la chloration des anilines se fait sur l'aniline libre, surtout quand le noyau porteur de la fonction amine est appauvri en électrons par des groupes électroattracteurs.

L'acide chlorhydrique dégagé par la chloration de l'aniline doit ultérieurement être neutralisée pour relibérer l'amine.

Le problème est particulièrement aigu dans le cas des anilines qui portent sur une chaîne latérale un carbone d'hybridation sp³ perhalogéné, et notamment perfluoré.

C'est pourquoi un des buts de la présente invention est de fournir un procédé qui comporte une étape d'échange chlore/fluor en milieu acide fluorhydrique (avec ou sans diluant) et qui permette d'éviter une double libération de l'aniline une fois d'avec l'acide fluorhydrique et une autre fois d'avec l'acide chlorhydrique.

Un autre but de la présente invention est de fournir un procédé qui permette une bonne récupération de l'acide fluorhydrique lié aux anilines.

Un autre but de la présente invention est de fournir un procédé du type précédent qui permette une limitation des rejets salins du procédé.

Ces buts et d'autres qui apparaîtront par la suite sont atteints au moyen du procédé de synthèse d'une aniline chlorée sur le noyau et comportant au moins un atome de carbone d'hybridation sp³ à la fois perhalogéné et porteur d'un atome de fluor caractérisé par le fait qu'il comporte l'étape de chloration d'une aniline précurseur de l'aniline chlorée, cette réaction étant menée dans un milieu fluorhydrique susceptible d'échanger le chlore et le fluor en position benzylique ou sur un carbone porteur d'un chalcogène.

Par carbone perhalogéné, il faut entendre un carbone de nature sp³, ne portant pas d'hydrogène et comportant, outre son lien avec l'aryle ou le chalcogène, au plus 2, avantageusement au plus 1, radicaux, tous les autres atomes étant des halogènes ; lesdits radicaux sont avantageusement choisis parmi les groupes électroattracteurs et ce, surtout, quand il y en a 2.

La présente invention est particulièrement utile pour les anilines qui portent un groupe électroattracteur en position para de la fonction amine. Ce groupe électroattracteur est avantageusement un groupe électroattracteur par effet inducteur, et notamment les groupes perhalogénés sur le carbone en liaison avec le noyau aromatique de l'aniline.

Le milieu fluorhydrique peut être, soit de l'acide fluorhydrique liquide, soit un acide fluorhydrique dilué dans un solvant.

Ce milieu fluorhydrique peut être simplement constitué de l'amine complexée par plusieurs molécules d'acide fluorhydrique, en général de 2 à 5, le plus souvent 4. En général, le rapport HF/aniline est compris entre 2 et 10, avantageusement entre 4 et 8. Les limites supérieures ne présentent qu'un sens économique.

Quoique l'aniline puisse être faite in situ par décomposition de fluorure de carbamoyle, cette mise en oeuvre n'est pas préférée en raison des risques d'émission de fluorophosgène (COCl₂). Selon une mise en oeuvre préférée, on réalise l'éventuelle "libération" de l'amine de son fluorure de carbamoyle avant de procéder à la chloration.

Les solvants utilisés sont avantageusement des solvants connus pour permettre la chloration ionique (chloration par une espèce du type Cl⁺).

Parmi les solvants utilisables, on peut notamment citer les dérivés fluorés et les dérivés aromatiques chlorés tels que, par exemple, les monochlorobenzènes, dichlorobenzènes, trichlorobenzènes.

Selon la présente invention, il est possible de réaliser une monochloration ou surtout une dichloration, la monochloration intervenant en ortho, la dichloration étant essentiellement en ortho-ortho' de la fonction aniline.

La chloration intervient en général à une température comprise entre 0 et 150°C, avantageusement entre 20 et 120°C.

La double chloration intervient à des températures supérieures à l'ambiante et donne de bons résultats à une température comprise entre 70°C et 150°C, les résultats les plus satisfaisants sont obtenus entre 90°c et 140°C.

Toutefois, pour obtenir de très bons rendements il est souhaitable de se placer à une température supérieure ou égale à 100°C (2 chiffres significatifs), de préférence supérieure à 100°C. Pour améliorer encore le rendement et la sélectivité, il est souhaitable d'introduire le substrat dans une masse réactionnelle (y compris solvant) répondant aux contraintes de température ci-dessus. Lors de la chauffe, pour éviter l'effet défavorable des températures intermédiaires inférieures aux températures préférées, on peut même envisager le chauffage du substrat, en particulier, et des réactifs en général avant introduction.

En outre, il est souhaitable que le chlore soit introduit de manière que le milieu reste toujours chlorant, en d'autres termes la vitesse de consommation de l'agent chlorant (e. g. le chlore) est au plus égale à la vitesse d'introduction dans le milieu dudit agent chlorant. Par exemple, le fait d'introduire le chlore de manière qu'il soit en surstoechiométrie par rapport à la réaction souhaitée constitue un paramètre opératoire améliorant à la fois le rendement et la sélectivité. Cette surstoechiométrie est avantageusement réalisée pendant la quasi-totalité de la réaction.

Afin d'éviter des réactions parasites, il est souhaitable que dès que le barbotage de chlore cesse, l'on refroidisse brutalement le mélange réactionnel. Ce refroidissement est avantageusement réalisé au moins partiellement par l'élimination de l'acide fluorhydrique sous pression réduite.

Selon un mode avantageux de mise en oeuvre de la réaction, le substrat et le chlore sont introduits progressivement et simultanément sur un pied de solvant ou de masse réactionnelle, portés et maintenus à la bonne température.

La présence d'acide de Lewis, tel que le trifluorure de bore, quoique favorisant la réaction, n'est pas nécessaire. La quantité molaire d'éventuel acide de Lewis est avantageusement comprise entre 10⁻⁴ et 10⁻¹ par rapport au substrat.

Avantageusement la pression à laquelle la réaction est menée est, soit la pression atmosphérique, soit une pression supérieure à la pression atmosphérique, de préférence autogène.

L'agent chlorant est avantageusement le chlore, lequel est introduit dans le milieu réactionnel avantageusement sous la forme d'un gaz. Cette introduction est avantageusement réalisée par le barbotage dans le milieu réactionnel, cette introduction étant de préférence réalisée à un débit au moins égal à celui défini par celui du substrat, multiplié par la stoechiométrie de la réaction visée (en général 2 moles de chlore pour 1 mole de substrat).

Parmi les avantages de la présente invention, il convient notamment de citer le gain d'une étape réactionnelle et d'une étape de neutralisation.

On peut également indiquer un rejet de sel significativement moins important et donc un meilleur respect de l'environnement. En effet, en milieu acide fluorhydrique, l'acide chlorhydrique se dégage. Il peut alors servir à la régénération du chlore soit par le procédé Deacon, soit par électrolyse.

L'acide chlorhydrique, notamment pour les petites installations, peut également servir à régénérer le chlore par action sur un hypochlorite. Toutefois, dans ce cas-là, le rejet de dérivés salins est plus important que dans le cas du procédé Deacon ou du procédé par électrolyse.

Un autre intérêt de la présente invention est de rendre beaucoup plus facile la libération de l'aniline de ses complexes hydrogénofluorés permettant ainsi un meilleur recyclage de l'acide fluorhydrique et un gain supplémentaire au niveau des rejets salins.

En effet, au cours de l'étude qui a conduit à la présente invention, il a été montré qu'une simple distillation de(s) acide(s) permettait d'éliminer l'acidité liée à l'aniline ainsi appauvrie par la chloration.

Ainsi lorsque la réaction est menée sous pression atmosphérique, l'acide chlorhydrique et l'acide fluorhydrique passe en phase gazeuse à partir de 20°C environ. Dans ces conditions, la libération de l'aniline est quasi-totale par distillation ou tout autre moyen de déplacer par passage en phase vapeur les acides fluorhydrique et chlorhydrique.

Cette libération peut, bien entendu, être menée sous pression supérieure à la pression atmosphérique, auquel cas la température de libération de l'aniline a lieu à une température plus élevée. Elle peut être menée sous pression réduite auquel cas la température de libération intervient à une température inférieure.

Ainsi la chloration des anilines visées par la présente invention permet une libération complète, aisée et non productrice de sels dans des complexes aniline-acide fluorhydrique.

Cette plus grande facilité à libérer l'aniline de ses complexes fluorhydriques est liée, d'une part, à l'augmentation du point d'ébullition de l'aniline chlorée par rapport à l'aniline non chlorée et d'autre part, par un amoindrissement de la basicité de l'aniline, ce qui dans ce cas semble affaiblir la liaison fluorhydrique aniline.

Cette libération de l'aniline est également facilitée par la plus grande stabilité de l'aniline chlorée par rapport à l'aniline de départ avant chloration.

Ces avantages sont particulièrement nets dans le cas du précurseur du "Fipronil" qu'est la paratrifluorométhylaniline (pTFMA) chlorée en ortho-ortho' de la fonction aniline.

En effet, dans les techniques précédentes, la réaction de chloration était réalisée sur la paratrifluorométhylaniline libérée de son acide fluorhydrique.

Les substrats bien adaptés à la présente invention sont les substrats répondant à la formule générale suivante.

Les substrats utilisables selon la présente invention répondent avantageusement à la formule :

(R)ₘ - Ar(-Z- (CX₂)ₚ-GEA)-NH-CO-F (forme fluorure de carbamoyle)

ou

(R)ₘ - Ar(-Z- (CX₂)ₚ-GEA)-NH-CO-F (NH₂) (forme aniline)

où :
□ Ar est un noyau aromatique, de préférence homocyclique ;
□ les X semblables ou différents représentent un fluor ou un radical de formule CₙF₂ₙ₊₁ avec n entier au plus égal à 5, de préférence à 2 ;
□ p représente un entier au plus égal à 2 ;
□ GEA représente un groupe hydrocarboné, un groupe électroattracteur dont les éventuelles fonctions sont inertes dans les conditions de la réaction, avantageusement fluor ou un reste perfluoré de formule CₙF₂ₙ₊₁ avec un entier au plus égal à 8, avantageusement à 5 ;
□ Le nombre total de carbones de -(CX₂)ₚ-GEA est avantageusement compris entre 1 et 15, de préférence entre 1 et 10.
□ m est 0 ou un entier choisi dans l'intervalle fermé (c'est-à-dire comprenant les bornes) 1 à 4 ;
□ R est un substituant inerte dans les conditions opératoires, avantageusement choisi parmi les halogènes avantageusement légers (c'est-à-dire chlore et fluor) et les radicaux hydrocarbonés, de préférence alcoyle, aryle, alcoylchalcogényle (tel que alcoyloxyle), arylchalcogényle (tel que aryloxyle) ;
□ Z représente une liaison simple, un atome de chalcogène avantageusement léger (soufre et oxygène).
En particulier, le substrat sous la forme aniline peut répondre à la formule : 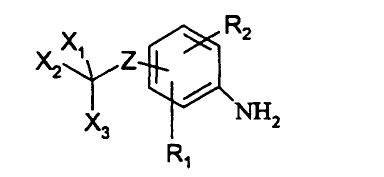 où :
□ Z représente une simple liaison ou un atome de chalcogène, où X₁, X₂, et parfois X₃ représentent des halogènes semblables et différents, avec la condition qu'au moins deux halogènes soient différents du fluor ;
□ R₁ et R₂ sont des substituants parmi les halogènes, les alcoyles, les aryles, les nitriles ;
□ Le radical X₃ peut être un groupe électroattracteur n'interférant pas avec la réaction et notamment peut être un groupe perfluoré, généralement désigné dans le domaine de la technique R_{f}.

La présente invention est notamment particulièrement intéressante pour la monochloration et la dichloration des trifluorométhylanilines et, en particulier, pour la mono- et surtout la dichloration de la paratrifluorochloroaniline.

Il est ainsi particulièrement intéressant d'utiliser la méthode qui vient d'être décrite ci-dessus pour la synthèse de la dichloroortho-ortho'-paratrifluorométhylaniline.

Cette technique ouvre une voie à la synthèse d'un N-phénylpyrazole, dichloré en position ortho-ortho' du pyrazole et portant en position para un groupe électroattracteur, avantageusement un atome de carbone d'hybridation sp³ à la fois perhalogéné et porteur d'un atome de fluor, comportant une étape de chloration d'une aniline selon l'invention. Dans le cas de dichloroparatrifluorométhylaniline il convient de mentionner qu'elle est, en particulier, utilisée pour réaliser, par des étapes en elles-mêmes connues, l'insecticide connu sous le nom de Fipronil.

Les exemples non limitatifs suivants illustrent l'invention :

### Exempte 1 : Chloration en milieu acide fluorhydrique de la pTFMA (paratrifluorométhylaniline)

On part d'une pTFMA associée à 5,5 molécules d'acide fluorhydrique. Ce produit est liquide. Dans un autoclave fermé, on introduit le produit de départ et 2,2 eqt de chlore par rapport à la pTFMA, c'est-à-dire 2,2 x de moles de chlore qu'il y a de pTFMA.
En autoclave fermé (aucun dégazage) avec 2,2 eqt de chlore % pTFMA.

Les résultats obtenus sont indiqués dans le tableau ci-dessous.

| N° essai | pTFMA, nHF | Cl2 | T° | durée | TT pTFMA | RR monochloro PTFMA | RR dichloro pTFMA | Remarque |
|---|---|---|---|---|---|---|---|---|
| 1 | n = 4,5 m ≅ 10 g | 2,2 eqt | 80°C | 5 h | 95% | 1,5% | 19% | |
| 2 | " | 2,6 eqt | 110°C | 6 h | 93% | 0,9% | 2,4% | |
| 3 | " +MCB ≅ 50% en poids | 2.2 eqt | 110°C | 6 h | 60% | 0,6% | 0,8% | |
| 4 | Pas de MCB | 2,1 eqt | 80°C | 5 h | 93,5% | 1,2% | 25% | |
| MCB signifie monochlorobenzène. | | | | | | | | |

Les résultats ainsi indiqués ne mesurent que les anilines présentes dans la phase fluorhydrique. En fait, l'analyse des résidus solides ou pâteux au fond du vaisseau ayant servi de réacteur montre qu'une bonne partie de la dichloroortho-ortho'paratrifluorométhylaniline se retrouve dans les résidus pâteux. En sorte que le rendement en dichloro est bien supérieur à celui qui est indiqué dans le tableau !

### Exemple 2 : essai en continu sous pression atmosphérique

Sur un pied (c'est-à-dire une masse se trouvant déjà dans le réacteur), porté à la température désirée, on introduit progressivement au moyen de 2 tubes plongeurs dont l'orifice est situé à proximité de la turbine d'agitation, d'une part le fluorhydrate de pTFMA (pTFMA-6,5 HF) et d'autre part le chlore.

La quantité de pTFMA introduite est de 1 mole. Les paramètres de fonctionnement et les résultats obtenus sont rassemblés dans le tableau suivant.

| N° essai | Température | Rapport molaire Cl₂/pTFMA | Durée d'introduction du chlore | Durée d'introduction du substrat | Rendement de réaction | Taux de transformation |
|---|---|---|---|---|---|---|
| 1 | 115°C ± 2 | 3,25 | 2 h | 2 h | ≥ 99% | 99,9% |
| 2 | 105°C ± 2 | 3 | 2 h | 1h ¾ | 86% | 100% |
| 3 | 105°C ± 2 | 2,35 | 2h 30 | 2 h | 82% | 100% |
| 4 | 80°C ± 2 | 2,3 | 1h 20 | 1h 20 | 45% | 98% |
| 5 | 110°C ± 2 | 2,5 | 3h | 3h | 94% | 99,5% |

Les résultats obtenus dans cet exemple, sont quelque peu meilleurs que ceux de l'exemple 1 en raison des réactions parasites pendant la montée en température.

## Revendications

1. Procédé de synthèse d'une aniline chlorée sur le noyau et comportant au moins un atome de carbone d'hybridation sp³ à la fois perhalogéné et porteur d'un atome de fluor, **caractérisé par le fait qu'**il comporte une étape de chloration d'une aniline précurseur de ladite aniline chlorée, ladite chloration étant réalisée dans un milieu fluorhydrique susceptible d'échanger le chlore et le fluor en position benzylique.

2. Procédé selon la revendication 1, **caractérisé par le fait que** ladite réaction est menée à une température comprise entre 0 et 150°C, avantageusement entre 20 et 120°C.

3. Procédé selon l'une des revendications 1 et 2, **caractérisé par le fait que** la réaction est menée en pression atmosphérique ou en pression autogène.

4. Procédé selon les revendications 1 à 3, **caractérisé par le fait que** l'on introduit le chlore progressivement par barbotage dans le milieu.

5. Procédé selon l'une des revendications 1 à 4, **caractérisé par le fait que** l'on introduit le chlore de manière que le milieu reste toujours chlorant.

6. Procédé selon l'une des revendications 1 à 5, **caractérisé par le fait que** dès que le barbotage de chlore cesse, on refroidit brutalement le mélange réactionnel.

7. Procédé selon l'une des revendications 1 à 6, **caractérisé par le fait que** ledit **caractérisé par le fait que** ledit refroidissement est réalisé, au moins partiellement, par élimination de l'acide fluorhydrique sous pression réduite.

8. Procédé selon l'une des revendications 1 à 7, **caractérisé par le fait que** l'aniline précurseur de ladite aniline chlorée est la p-trifluorométhylaniline.

9. Procédé de synthèse d'un N-phénylpyrazole dichloré, en position ortho-ortho' du pyrazole, et portant en position para un groupe électroattracteur, avantageusement un atome de carbone d'hybridation sp³ à la fois perhalogéné et porteur d'un atome de fluor, **caractérisé par le fait qu'**il comporte une étape d'une aniline chlorée selon la revendication 1.

## Patentansprüche

1. Verfahren zur Synthese eines Anilins, chloriert am Kern und umfassend mindestens ein Kohlenstoffatom der Hybridisierung sp³, gleichzeitig perhalogeniert und Träger eines Fluoratoms, **dadurch gekennzeichnet, daß** es eine Stufe der Chlorierung eines Anilins umfaßt, das Vorläufer des genannten chlorierten Anilins ist, wobei die genannte Chlorierung in einem Medium von Fluorwasserstoffsäure realisiert wird, das fähig ist, das Chlor und das Fluor in Benzylposition auszutauschen.

2. Verfahren nach Anspruch 1, **dadurch gekennzeichnet, daß** die genannte Reaktion bei einer Temperatur einschließlich zwischen 0 °C und 150 °C, vorteilhafterweise zwischen 20 °C und 120 °C durchgeführt wird.

3. Verfahren nach einem der Ansprüche 1 und 2, **dadurch gekennzeichnet, daß** die Reaktion unter atmosphärischem Druck oder unter autogenem Druck durchgeführt wird.

4. Verfahren nach Anspruch 1 bis 3, **dadurch gekennzeichnet, daß** man das Chlor nach und nach durch Einleiten in das Medium einträgt.

5. Verfahren nach einem der Ansprüche 1 bis 4, **dadurch gekennzeichnet, daß** man das Chlor in der Weise einträgt, daß das Medium immer chlorierend bleibt.

6. Verfahren nach einem der Ansprüche 1 bis 5, **dadurch gekennzeichnet, daß** man, sobald das Einleiten von Chlor aufhört, die Reaktionsmischung stark abkühlt.

7. Verfahren nach einem der Ansprüche 1 bis 6, **dadurch gekennzeichnet, daß** das genannte Abkühlen mindestens teilweise durch Entfernung der Fluorwasserstoffsäure unter reduziertem Druck realisiert wird.

8. Verfahren nach einem der Ansprüche 1 bis 7, **dadurch gekennzeichnet, daß** das Vorläufer des genannten chlorierten Anilins ist p-trifluoromethylanilin.

9. Verfahren zur Synthese eines in Position ortho-ortho' des Pyrazols dichlorierten N-Phenylpyrazols, das in Position para eine elektronenanziehende Gruppe trägt, vorteilhafterweise ein Kohlenstoffatom der Hybridisierung sp³, gleichzeitig perhalogeniert und Träger eines Fluoratoms, **dadurch gekennzeichnet, daß** es eine Stufe eines chlorierten Anilins nach Anspruch 1 umfaßt.

## Claims

1. Process for the synthesis of a chlorinated aniline which is chlorinated on the ring and which comprises at least one carbon atom of sp³ hybridization which is both perhalogenated and the carrier of a fluorine atom, **characterized in that** it comprises a stage of chlorination of a precursor aniline of said chlorinated aniline, said chlorination being carried out in a hydrofluoric acid medium capable of exchanging chlorine and fluorine in the benzyl position.

2. Process according to Claim 1, **characterized in that** said reaction is carried out at a temperature of between 0 and 150°C, advantageously between 20 and 120°C.

3. Process according to either of Claims 1 and 2, **characterized in that** the reaction is carried out at atmospheric pressure or under autogenous pressure.

4. Process according to Claims 1 to 3, **characterized in that** the chlorine is gradually introduced by sparging into the medium.

5. Process according to one of Claims 1 to 4, **characterized in that** the chlorine is introduced so that the medium always remains chlorinating.

6. Process according to one of Claims 1 to 5, **characterized in that**, as soon as sparging of chlorine ceases, the reaction mixture is suddenly cooled.

7. Process according to one of Claims 1 to 6, **characterized in that** said cooling is carried out, at least partially, by removal of the hydrofluoric acid under reduced pressure.

8. Process according to one of Claims 1 to 7, **characterized in that** the precursor aniline of said chlorinated aniline is p-trifluoromethylaniline.

9. Process for the synthesis of an N-phenylpyrazole, dichlorinated in the position ortho-ortho' to the pyrazole and carrying, in the para position, an electron-withdrawing group, advantageously a carbon atom of sp³ hybridization which is both perhalogenated and the carrier of a fluorine atom, **characterized in that** it comprises a stage of a chlorinated aniline according to Claim 1.
